# EUROPEAN PATENT SPECIFICATION

(11) **EP 1 001 744 B1**
(45) Date of publication and mention of the grant of the patent: **09.11.2005**
(21) Application number: 98932505.5
(22) Date of filing: 09.07.1998
(51) Int. Cl.: A61K 9/00

(54) **MICROPARTICLES BASED ON HYBRID POLYMERIC MATERIALS FOR CONTROLLED RELEASE OF BIOLOGICALLY ACTIVE MOLECULES, A PROCESS FOR PREPARING THE SAME AND THEIR USES FOR $i(IN VIVO) AND $i(IN VITRO) THERAPY, PROPHYLAXIS AND DIAGNOSTICS**
MIKROPARTIKEL AUF DER GRUNDLAGE VON HYBRIDPOLYMERMATERIALEN ZUR KONTROLLIERTEN ABGABE VON BIOLOGISCH AKTIVEN MOLEKÜLEN, VERFAHREN ZU IHRER HERSTELLUNG UND DEREN VERWENDUNG IN DER IN-VIVO ODER IN-VITROTHERAPIE, PROPHYLAXE UND DIAGNOSTIK
MICROPARTICULES A BASE DE MATERIAUX POLYMERES HYBRIDES PERMETTANT DE LIBERER DE MANIERE REGULEE DES MOLECULES ACTIVES SUR LE PLAN BIOLOGIQUE, PROCEDE DE PREPARATION DE CES MICROPARTICULES, ET UTILISATION DE CES DERNIERES $i(IN VIVO) ET $i(IN VITRO) LORS DE THERAPIES, DE TRAITEMENTS PREVENTIFS ET DE DIAGNOSTIQUE

(30) Priority: 10.07.1997 IT RM970417
(43) Date of publication of application: 24.05.2000
(73) Proprietor: ISTITUTO SIEROVACCINOGENO ITALIANO I.S.I. S.p.A., 55020 Castelvecchio Pascoli (Lucca) (IT)
(72) Inventor: COWDALL, Jenny, Shrewsbury, Shropshire SY3 0NZ (GB); DAVIES, John, Shrewsbury, Shropshire SY3 0NZ (GB); ROBERTS, Mark, Shrewsbury, Shropshire (GB); CARLSSON, Anders, S-112 38 Stockholm (SE); SOLARO, Roberto, I-56100 Pisa (IT); MAZZANTI, Giuseppe, I-55020 Castelvecchio Pascoli (IT); CHIELLINI, Elisabetta, I-56100 Pisa (IT); CHIELLINI, Emo, I-56100 Pisa (IT); SÖDERLIND, Erik, S-431 37 Mölndal (SE)
(74) Representative: Gervasi, Gemma
(86) International application number: PCT/IT1998/000191
(87) International publication number: WO 1999/002131

(56) References cited:
- WO-A1-93/13753
- WO-A1-96/10992
- DE-A1- 1 951 365
- US-A- 5 102 872
- Macromol. Rapid Commum., volume 15, 1994, Marcela Brissová et al, "Reaction of maleic anhydride copolymers with albumin in water systems", page 211 - page 216, XP002900305
- Art. Cells, Blood Subs., and Immob. Biotech, Volume 24, No. 3, 1996, Y. Murat Elcin et al, "Controlled Release of Endothelial Cell Growth Factor From Chitosan-Albumin Microspheres for Localized Angiogenesis: In Vitro and In Vivo Studies" page 257 - page 271, XP002900306
- J. Biomater Sci. Polymer Edin, Volume 7, No. 12, 1996, Terence W. Atkins et al, "The incorporation and release of bovine serum albumin from poly (Beta-hydroxybutyratehydroxyvalerate) microspheres", page 1065 - page 1073, XP002900307
- STN International, File CAPLUS, CAPLUS accession no. 1994:541505, Mueller, B.W et al: "Microparticle production without organic solvents", XP002900308 & Minutes Int. Symp. Microencapsulation, 9th (1994), Meeting Date 1993, 29-40

## Description

This invention relates to microparticles based on hybrid polymeric materials for controlled release of biologically active molecules, to a process for preparing said microparticles and to their uses for *in vitro* and *in vivo* therapy, prophylaxis and diagnostics.

More particularly, this invention relates to the preparation of chemically homogeneous microparticles consisting of a component of proteic kind and of a natural, synthetic, semisynthetic polymer.

Prior art mentions numerous examples of preparation (and of employment) of macromolecular aggregates and of microparticles which are made up of polymeric materials of proteic or non-proteic nature, synthetic and semisynthetic, for instance cf. Carth W. Hastings and Paul Ducheyne, Macromolecular Biomaterials, CRC Press Inc. (1984).

Prior art also mentions examples of preparation of macroaggregates and microparticles containing both proteins (and polypeptides) and natural, synthetic, semisynthetic polymers as well.

Among proteins, a particular emphasis has been put on albumin, as it interacts with many compounds, and among them the compounds containing electronegative functional groups or acid groups.

In addition to its reaction with long-chain at acids, Carr C.W., Proc. Soc. Expl. Biol. Med. 89: 546 (1959), Bears A.G., Am. J. Med. 22: 747 (1957), albumin also interacts with: anionic polyelectrolytes such as heparin, H.F.M. Cremers et al., J. Controlled Rel. 11, 167:179 (1990), polyelectrolytes deriving from copolymerization of maleic anhydride and vinyl ethers or vinyl esters, H. Ohno et al., Makromol. Chem. 182: 1253 (1981); M. Brissova, Makromol. Rapid Comm. 15: 211 - 216 (1994), with cationic polyelectrolytes such as chitosan, Y. Murat Elcin, Art. Cells Blood. Subs. & Immob. Biotech. 24 (3) 257 - 271 (1996).

Prior art also mentions numerous examples of incorporation of proteins and polypeptides into natural and artificial polymeric matrices. However, the incorporation of albumin (human, bovine) has been finalized exclusively to analytical purposes, T. Atkins, J. Biomat. Sci. Polymer Edn. 7: 1065 - 1073 (1996), for modulating the release of bioactive protein molecules, U.S. 5.102.872, or as a stabilizing agent for proteins, M.A. Hanson, Introduction to Formulation of Protein Pharmaceutical, Plenum Press 1992, pg. 209, as a compound suitable for stabilizing microparticles as to phagocytosis exerted by polymorphonucleate cells, T. Verrecchia, J. Control Rel. 36: 49 - 51 (1995).

The International Patent Application WO 96/10992 discloses microsphers comprising protein products and at least one alfa-hydroxy acid (p.5, last par.).

Murat Elcin et al. (1996, Art. Cells, Blood Subs., and Immob. Biotech., 24(3), 257-271) refers in general to microspheres made of a polysaccharide, chitosan and albumin (see abstract).

Atkins and Peacock (1996, J. Biomatec. Sci. Polymer Edn. Vol. 7, N. 12, 1065-1073) discloses microspheres composed by a biodegradable polymer and proteins. However the preparation process comprises a *"single emulsion technique with solvent evaporation*" (see abstract).

However, in all cases mentioned above the microspheres are obtained by interaction of proteins with polymers of hydrophobic nature. This brings about the use of organic solvents for dissolving the synthetic polymer, with the consequent at least partial denaturation of proteins. Moreover, microspheres so obtained are made up of a heterogeneous aggregation of proteic material and synthetic polymer, in general of a proteic mass coated by a hydrophobic synthetic polymer shell.

The Authors of this invention have discovered that by suitably mixing solutions containing at least a water soluble protein and water solutions containing variable amounts of organic solvents that contain at least one anionic, natural, synthetic, semisynthetic polyelectrolyte under particular and definite mechanical and environmental conditions, chemically homogeneous microparticles are obtained, which are advantageously employable for controlled release of biologically active molecules, for both *in vitro* and *in vivo* therapy, prophylaxis and diagnostics, and in the cosmetic and dietetic fields as well.

Numerous methods for the preparation of microparticles are known. J. Ogawa, J. Biomater. Sci. Polym. EDN 8: 391 - 409 (1997). All known methods are based essentially on phase separation, solvent evaporation, spray-drying, emulsification, interface polymerisation. However, no methods already known provide the employment of analogous techniques similar or equal to that which is the object of this invention, and they do not result in chemically homogeneous particles.

Accordingly, it is the object of this invention a microparticle which is chemically homogeneous and is made up of a proteic component and a natural, synthetic, or semisynthetic polymer or a mixture of such polymers.

Preferably, the proteic components comprises at least one protein from albumin, alpha 1-globulin, alpha 2-globulin, collagen, fibrinogen, gelatin, more preferably albumin is human albumin, natural or recombinant.

The polymers comprise semiesters of alternate copolymers of maleic anhydride with oligoethyleneglycol vinyl ethers whose oligomerization degree is 1 ≤ n ≤ 100, more preferably 1 ≤ n ≤ 10.

Preferably the microparticle is spheroidal in shape, with a diameter between 50 nm and 2,000 nm, more preferably between 100 nm and 300 nm.

It is a further object of this invention a process for the preparation of microparticles according to this invention, which process comprises the step of mixing a first solution containing the natural polymer at a concentration between 5 g and 200 g/l, with a mixture of hydrophilic, protic and aprotic organic solvents, with a second solution containing the proteic component at a concentration between 1 g and 40 g/l, in which the weight ratio of the polymer to the proteic component is between 100 and 1, at a pH between 2 and 9, and at a temperature between 0°C and 60°C, under stirring conditions so as to prevent aggregation phenomena from occurring and so as to favor coprecipitation of the polymer and of the proteic component.

Preferably the organic solvent of the first solution is made up of ethanol, alternatively of a water/ethanol mixture with an ethanol content between 0% and 80%.

Preferably the proteic component in the first solution is at a concentration between 2 g and 6 g/l.

Preferably the polymer in the second solution is at a concentration between 40 g and 60 g/l.

Preferably the weight ratio of the polymer to the proteic component is between 10 and 1, and more preferably between 5 and 1.

Preferably the pH is between 4 and 7.

Preferably, the temperature is comprised between 4°C and 25°C.

It is a further object of this invention the employment of the microparticles of this invention for therapeutic, prophylactic, dietetic, analytic, cosmetic and preparative objectives of systems featuring controlled release of active molecules.

This invention will be disclosed now by means of examples of the same, which are not limitative of its object and scope.

### Example 1

### Preparation of PAM14/HSA microspheres

A solution of 60 mg of methyl hemiester of the alternate copolymer of maleic anhydride/methoxyethyl vinyl ether (PAM14) in 2.0 ml of water was dropped by means of a syringe supplied with a 22G needle into a solution of 24 mg of human seroalbumin (HSA) in 2.0 ml of bidistilled water kept under magnetic stirring. When the addition was complete, magnetic stirring was kept for 60 minutes. Dimensional analysis by means of dynamic light scattering showed that the microspheres were characterized by an average diameter of 140 nm and by a polydispersion index of 0.1 - 0.3.

### Example 2

### Preparation of PAM11/MYO/HSA microspheres

A solution of 100 mg of the methyl hemiester of the alternate copolymer of maleic anhydride/methoxyethyl vinyl ether (PAM11) in 3.0 ml of a mixture 1:3 of ethanol/water was dropped by means of a syringe fitted with a 22G needle into a solution of 50 mg of a mixture 1:4 of myoglobin/human seroalbumin (MYO/HSA) in 8 ml of bidistilled water under magnetic stirring. After completing the addition, magnetic stirring was kept for a further time of 60 minutes. Dimensional analysis by means of dynamic light scattering showed that the microspheres were characterized by an average diameter of 450 nm and a polydispersion index of 0.1 - 0.3.

### Example 3

### Preparation of PAM14/HSA/HSA-FITC/MAN microspheres

A solution of 210 mg of the butyl hemiester of the alternate copolymer maleic anhydride/methoxyethyl vinyl ether (PAM14) in 4.0 ml of a 1:4 mixture of ethanol/water was dropped by means of a syringe fitted with a 22G needle into a solution of 40 mg of human seroalbumin (HSA) and 600 mg of human seroalbumin fluorescein isothiocyanate (HSA-FITC) in 10 ml of bidistilled water containing 10% wt. of mannitol (MAN). When the addition was finished, magnetic stirring was kept for 90 minutes. Dimensional analysis by means of dynamic light scattering showed that the microspheres were characterized by an average diameter of 160 nm and a polydispersion index of 0.1 - 0.3.

### Example 4

### Preparation of PAM14/HSA/GDA-bCD microspheres

A solution of 200 mg of butyl hemiester of the alternate copolymer maleic anhydride/methoxyethyl vinyl ether (PAM14) in 4.0 ml of a 3:2 ethanol/water mixture was dropped by means of a syringe fitted with a 22G needle into a solution of 40 mg of human seroalbumin (HSA) in 10.0 ml of bidistilled water containing 5% wt. glycidyldiiso-propylidenarabitol-betacyclodextrin (GDA-bCD). When the addition was finished, the dispersion was kept magnetically stirred for 60 minutes. Dimensional analysis by means of dynamic light scattering showed that the microspheres were characterized by an average diameter of 190 nm and by a polydispersion index of 0.1 - 0.2.

### Example 5

### Preparation of PAM14/HSA/ASFET microspheres

A solution of 100 mg of butyl hemiester of the alternate copolymer of maleic anhydride/methoxy vinyl ether (PAM14) in 2.0 ml of a 3:2 mixture of ethanol/water was dropped by means a syringe fitted with a 22G needle into a solution of 31 mg of human seroalbumin (HSA) and 11 mg of asialofetuin (ASFET) in 5.0 ml of bidistilled water. The dispersion of microsphere was left under magnetic stirring for 60 minutes. Dimensional analysis by means of dynamic light scattering showed that the microspheres were characterized by an average diameter of 1450 nm and a polydispersion index of 0.6.

### Example 6

### Preparation of microspheres of AA/HSA/GDX-bCD

A solution of 100 mg of polyacrylic acid (AA) in 4 ml of water was added by means of a syringe fitted with a 22G needle into a solution of 60 mg of human seroalbumin (HSA) in 10 ml of bidistilled water containing 5% by weight of glycidyldiisopropylidenxylitolbetacyclodextrin (GDA-bCD), under magnetic stirring. When the addition was finished, magnetic stirring was kept for a further time of 60 minutes. Dimensional analysis by means of dynamic light scattering showed that the microspheres were characterized by an average diameter of 350 nm and a polydispersion index of 0.3 - 0.5.

### Example 7

### Preparation of microspheres of AAL/HSA/GLU

A solution of 200 mg of alginic acid (AAL) in 4 ml of water was dropped by means of a syringe fitted with a 22G needle into a solution of 60 mg of human seroalbumin (HSA) in 12 ml of bidistilled water containing 7% by weight of glucose, under magnetic stirring. Magnetic stirring was kept going on for 60 minutes. Dimensional analysis by means of dynamic light scattering showed that the microspheres were characterized by an average diameter of 210 nm and a polydispersion index of 0.2 - 0.4.

## Claims

1. A process for the preparation of chemically homogeneous microparticles consisting of a first component having a proteic origin and of a second component being natural, synthetic or semisynthetic poly-anionic polymers or of a mixture of them, said process comprising the step of mixing a first solution containing said polymer at a concentration between 5 g and 200 g per litre with a mixture of hydrophilic protic and aprotic organic solvents, with a second solution containing the proteic component at a concentration between 1 g and 40 g per litre, wherein the weight ratio of the polymer to the proteic component is between 100 and 1, at a pH between 2 and 9, and at a temperature between 0°C and 60°C, under such conditions of stirring as to prevent aggregation phenomena from occurring and as to favor the coprecipitation of the polymer and of the proteic component.

2. A process according to claim 1, wherein the organic solvent of the first solution is ethanol.

3. A process according to claim 2, wherein the organic solvent of the first solution is made up of a water/ethanol mixture with an ethanol content between 0% and 80%.

4. A process according to claim 3, wherein the proteic component in the first solution is at a concentration between 2 g and 6 g per litre.

5. A process according to claim 4, wherein the polymer in the second solution is at a concentration between 40 g and 60 g per litre.

6. A process according to claim 1, wherein the weight ratio of the polymer to the proteic component is between 10 and 1.

7. A process according to claim 6, wherein the weight ratio of the polymer to the proteic component is between 5 and 1.

8. A process according to claim 1, wherein the pH is between 4 and 7.

9. A process according to claim 1, wherein the temperature is between 4°C and 25°C.

10. A chemically homogeneous microparticle consisting of a first component having a proteic origin and of a second component being a semiester of alternate copolymers of maleic anhydride with oligoethyleneglycol vinyl esters whose oligomerisation degree is 1 ≤ n ≤ 100.

11. A chemically homogeneous microparticle according to claim 10 wherein the oligomerisation degree of said second component is 1 ≤ n ≤ 10.

12. A chemically homogeneous microparticle according to claim 10, wherein the proteic component comprises at least a protein chosen from among albumin, alpha 1-globulin, alpha 2-globulin, collagen, fibrinogen, gelatin.

13. A chemically homogeneous microparticle according to claim 12, wherein said protein is natural or recombinant human albumin.

14. A chemically homogeneous microparticle according to any of claims from 10 to 13, having a spheroidal shape and has a diameter between 50 nm and 2000 nm.

15. A chemically homogeneous microparticle according to claim 14, wherein the diameter is between 100 nm and 300 nm.

16. Use of the microparticles according to anyone of the preceding claims 10-15, for the preparation of a therapeutic, diagnostic, prophylactic, dietetic, analytic or cosmetic preparation.

17. Use of the microparticles according to anyone of the preceding claims 10-15, for the preparation of active molecule controlled-release systems.

## Patentansprüche

1. Ein Verfahren zur Herstellung von chemisch homogenen Mikropartikeln bestehend aus einer ersten Komponente mit Proteinursprung und einer zweiten Komponente, die ein natürliches, synthetisches oder halbsynthetisches polyanionisches Polymer ist oder einer Mischung daraus; genanntes Verfahren enthaltend den Schritt der Mischung einer ersten Lösung, enthaltend genanntes Polymer in einer Konzentration zwischen 5 g und 200 g pro Liter mit einer Mischung aus hydrophilen protischen und aprotischen organischen Lösungsmitteln, mit einer zweiten Lösung enthaltend die Protein-Komponente in einer Konzentration zwischen 1 g und 40 g pro Liter, wobei das Gewichtsverhältnis von Polymer zu Protein-Komponente zwischen 100 und 1 liegt, bei einem pH-Wert zwischen 2 und 9 und einer Temperatur zwischen 0°C und 60°C und unter solchen Bedingungen gerührt wird, um das Aggregationsphänomen zu verhindern und die Kopräzipitation von Polymer und Protein-Komponente zu begünstigen.

2. Verfahren gemäß Anspruch 1, wobei das organische Lösungsmittel in der ersten Lösung Ethanol ist.

3. Verfahren gemäß Anspruch 2, wobei das organische Lösungsmittel in der ersten Lösung aus einer Wasser/Ethanol-Mischung besteht mit einem Ethanol-Gehalt zwischen 0% und 80%.

4. Verfahren gemäß Anspruch 3, wobei die Proteinkomponente in der ersten Lösung bei einer Konzentration von 2 g bis 6 g pro Liter liegt.

5. Verfahren gemäß Anspruch 4, wobei das Polymer in der zweiten Lösung bei einer Konzentration von 40 g bis 60 g pro Liter liegt.

6. Verfahren gemäß Anspruch 1, wobei das Gewichtsverhältnis von Polymer zu Proteinkomponente zwischen 10 und 1 beträgt.

7. Verfahren gemäß Anspruch 6, wobei das Gewichtsverhältnis von Polymer zu Proteinkomponente zwischen 5 und 1 beträgt.

8. Verfahren gemäß Anspruch 1, wobei der pH-Wert zwischen 4 und 7 liegt.

9. Verfahren gemäß Anspruch 1, wobei die Temperatur zwischen 4°C und 25°C beträgt.

10. Chemisch homogene Mikropartikel bestehend aus einer ersten Komponente mit Proteinursprung und einer zweiten Komponente, die ein Halbester der alternierenden Kopolymere von Maleinanhydrid mit Oligoethylenglycolvinylestern und einem Oligomerisationsgrad von 1 ≤ n ≤ 100 ist.

11. Chemisch homogene Mikropartikel gemäß Anspruch 10, wobei der Oligomerisationsgrad der genannten zweiten Komponente bei 1 ≤ n ≤ 10 liegt.

12. Chemisch homogene Mikropartikel gemäß Anspruch 10, wobei die Proteinkomponente mindestens ein Protein umfasst, ausgewählt unter Albumin, Alpha 1-Globulin, Alpha 2-Globulin, Kollagen, Fibrinogen, Gelatine.

13. Chemisch homogene Mikropartikel gemäß Anspruch 12, wobei es sich bei dem genannten Protein um natürliches oder rekombinantes Human-Albumin handelt.

14. Chemisch homogene Mikropartikel gemäß einem der Ansprüche von 10 bis 13 in Kugelform und mit einem Durchmesser zwischen 50 nm und 2000 nm

15. Chemisch homogene Mikropartikel gemäß Anspruch 14, welche einen Durchmesser zwischen 100 nm und 300 nm besitzen.

16. Verwendung von Mikropartikeln gemäß einem der vorherigen Ansprüche 10-15 zur Herstellung von therapeutischen, diagnostischen, prophylaktischen, diätetischen, analytischen oder kosmetischen Zubereitungen.

17. Verwendung von Mikropartikeln gemäß einem der vorherigen Ansprüche 10-15 zur Herstellung von aktivem Molekül mit kontrolliertem Freisetzungssystem.

## Revendications

1. Procédé de préparation de microparticules chimiquement homogènes consistant en un premier composant ayant une origine protéique et en un second composant qui est des polymères poly-anioniques naturels, synthétiques ou semisynthétiques ou en un mélange de ceux-ci, ledit procédé comprenant l'étape consistant à mélanger une première solution contenant ledit polymère à une concentration comprise entre 5 g et 200 g par litre avec un mélange de solvants organiques protiques et aprotiques hydrophiles, avec une seconde solution contenant le composant protéique à une concentration comprise entre 1 g et 40 g par litre, dans lequel le rapport en poids du polymère sur le composant protéique est compris entre 100 et 1, à un pH compris entre 2 et 9, et à une température comprise entre 0° C et 60° C, dans des conditions d'agitation telles à empêcher que des phénomènes d'aggrégation ne se produisent et à favoriser la coprécipitation du polymère et du composant protéique.

2. Procédé selon la revendication 1, dans lequel le solvant organique de la première solution est l'éthanol.

3. Procédé selon la revendication 2, dans lequel le solvant organique de la première solution est constitué d'un mélange d'eau/éthanol avec une teneur en éthanol comprise entre 0 % et 80 %.

4. Procédé selon la revendication 3, dans lequel le composant protéique dans la première solution est à une concentration comprise entre 2 g et 6 g par litre.

5. Procédé selon la revendication 4, dans lequel le polymère dans la seconde solution est à une concentration comprise entre 40 g et 60 g par litre.

6. Procédé selon la revendication 1, dans lequel le rapport en poids du polymère sur le composant protéique est compris entre 10 et 1.

7. Procédé selon la revendication 6, dans lequel le rapport en poids du polymère sur le composant protéique est compris entre 5 et 1.

8. Procédé selon la revendication 1, dans lequel le pH est compris entre 4 et 7.

9. Procédé selon la revendication 1, dans lequel la température est comprise entre 4° C et 25° C.

10. Microparticule chimiquement homogène consistant en un premier composant ayant une origine protéique et en un second composant qui est un semiester de copolymères alternés d'anhydride maléique avec des esters d'oligoéthylèneglycol vinyle dont le degré d'oligomérisation est 1 ≤ n ≤ 100.

11. Microparticule chimiquement homogène selon la revendication 10, dans laquelle le degré d'oligomérisation dudit second composant est 1 ≤ n ≤ 10.

12. Microparticule chimiquement homogène selon la revendication 10, dans laquelle le composant protéique comprend au moins une protéine choisie parmi l'albumine, l'alpha 1-globuline, l'alpha 2-globuline, le collagène, le fibrinogène et la gélatine.

13. Microparticule chimiquement homogène selon la revendication 12, dans laquelle ladite protéine est l'albumine humaine naturelle ou recombinante.

14. Microparticule chimiquement homogène selon l'une quelconque des revendications 10 à 13, ayant une forme sphéroïdale et un diamètre compris entre 50 nm et 2 000 nm.

15. Microparticule chimiquement homogène selon la revendication 14, dans laquelle le diamètre est compris entre 100 nm et 300 nm.

16. Utilisation des microparticules selon l'une quelconque des revendications précédentes 10 à 15, pour la préparation d'une préparation thérapeutique, diagnostique, prophylactique, diététique, analytique ou cosmétique.

17. Utilisation des microparticules selon l'une quelconque des revendications précédentes 10 à 15, pour la préparation de systèmes de libération contrôlée de molécules actives.
